# EUROPÄISCHE PATENTSCHRIFT

(11) **EP 0 688 766 B1**
(45) Veröffentlichungstag und Bekanntmachung des Hinweises auf die Patenterteilung: **24.03.1999**
(21) Anmeldenummer: 95109203.0
(22) Anmeldetag: 14.06.1995
(51) Int. Cl.: C07C 279/22, A61K 31/16

(54) **Perfluoralkylgruppen tragende phenylsubstituierte Alkenylcarbonsäure-guanidide, Verfahren zu ihrer Herstellung, ihre Verwendung als Medikament oder Diagnostikum sowie sie enthaltendes Medikament**
Phenylsubstituted alkenyl-carboxylic-acid-guanidines containing perfluoroalkyl groups, process for their preparation, their use as medicament or diagnostic agent, as well as a medicament containing them
Guanidines d'acides alcénylcarboxyliques substituées par un groupe phényle portant des groupes perfluoroalcoyle, procédé pour leur préparation, leur utilisation comme médicament ou agent diagnostique, ainsi que médicament les contenant

(30) Priorität: 20.06.1994 DE 4421536
(43) Veröffentlichungstag der Anmeldung: 27.12.1995
(73) Patentinhaber: HOECHST AKTIENGESELLSCHAFT, 65929 Frankfurt am Main (DE)
(72) Erfinder: Schwark, Jan-Robert, Dr., D-65929 Frankfurt (DE); Kleemann, Heinz-Werner, Dr., D-65474 Bischofsheim (DE); Lang, Hans-Jochen, Dr., D-65719 Hofheim (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 612 723
- EP-A- 0 628 543
- WO-A-84/00875

## Beschreibung

Die Erfindung betrifft Perfluoralkylgruppen tragende phenylsubstituierte Alkenylcarbonsäure-guanidide der Formel I 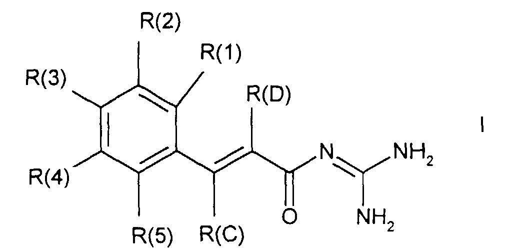 worin bedeuten:
- R(C): Wasserstoff, F, Cl, Br, I, CN, (C₁-C₈)-Alkyl_{,} C_{b}F_{2b+1};
b 1, 2, 3, 4, 5, 6, 7 oder 8;
- R(D): unabhängig wie R(C) definiert,
- R(1): Wasserstoff, (C₁-C₄)-Alkyl, C_{b}F_{2b+1}, (C₃-C₈)-Cycloalkyl, F, Cl, Br, I oder CN;
b 1, 2, 3 oder 4;
- R(2), R(3), R(4) und R(5): unabhängig voneinander wie R(1) definiert;
jedoch unter der Bedingung,
daß mindestens einer der Substituenten R(C), R(D), R(1), R(2), R(4) oder R(5) eine C_{b}F_{2b+1}-Gruppe, und daß R(3) keine C_{b}F_{2b+1}-Gruppe ist.

Enthält die Verbindung der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Doppelbindungsgeometrie der Verbindungen der Formel I kann sowohl E als auch Z sein. Die Verbindungen können als Doppelbindungsisomere im Gemisch vorliegen.

Die bezeichneten Alkyl- und Perfluoralkyl-reste können sowohl geradkettig als auch verzweigt vorliegen.

Die Erfindung betrifft weiterhin ein Verfahren zur Herstellung der Verbindung I, dadurch gekennzeichnet, daß man Verbindungen der Formel II 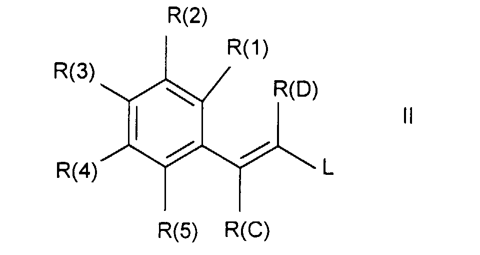 mit Guanidin umsetzt, worin R(1) bis R(5) und R(A), R(B), R(C) und R(D) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetra fluoborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Carbonsäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigem Guanidin zur Abbindung der Halogenwasserstoffsäure.

Ein Teil der zugrundeliegenden Carbonsäurederivate der Formel II ist bekannt und in der Literatur beschrieben. Die unbekannten Verbindungen der Formel II können nach literaturbekannten Methoden hergestellt werden. Die erhaltenen Alkenylcarbonsäuren werden nach einer der oben beschriebenen Verfahrensvarianten zu erfindungsgemäßen Verbindungen I umgesetzt.

Die Einführung einiger Substituenten gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. Organozink-verbindungen.

Carbonsäureguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 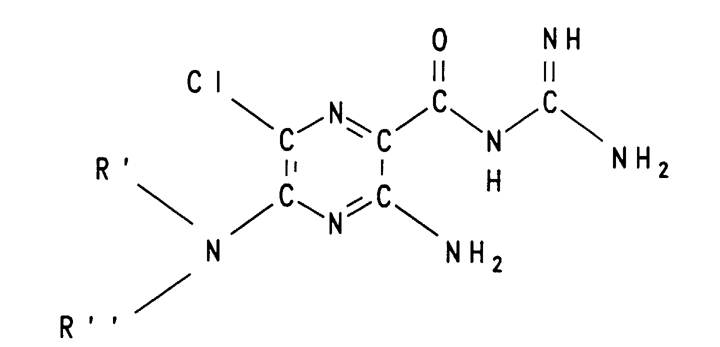 Amilorid: R', R'' = H
Dimethylamilorid: R`, R`` = CH₃
Ethylisopropylamilorid: R' = C₂H₅, R`` = CH(CH₃)₂

Darüberhinaus sind Untersuchungen bekannt geworden, die auf antiarrhythmische Eigenschaften von Amilorid hinweisen [Circulation 79, 1257-63 (1989)]. Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988), book of abstracts]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Die PCT-Veröffentlichung WO 84 00875 bezieht sich auf Guanidin-Verbindungen, die auch Benzoylgruppen und eine ethylenische Gruppe zwischen dem Phenylkern und der Guanidylgruppe enthalten können; jedoch ist stets eine in allen praktischen Ausführungen im Guanidin-Teil des Moleküls stets eine Alkylgruppe enthalten, es handelt sich in keinem Falle um in der Guanidylgruppe unsubstituierte Verbindungen der erfindungsgemäßen Struktur. Außerdem enthält keine der tatsächlich hergestellten oder speziell vorgeschlagenen Verbindungen eine Perfluoralkyl-Gruppe. Verwendet werden diese bekannten Verbindungen zum Behandeln von Infektionen, die auf Protozoen zurückgehen. Die Stoffansprüche in den "amended claims" sind auch ausschließlich auf solche alkylierten Verbindungen gerichtet (mit R4 nach der Definition der WO 84-00 875 als lower alkyl).

Die EP 628 543 und die EP 612 723 schlagen reine Benzoylguanidine vor, bei denen in keinem Falle eine ungesättigte Gruppe zwischen dem Phenylkern und der Acylguanidin-Gruppe enthalten ist.

Im US-Patent 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet. Auch ier werden keine Verbindungen mit einer ungesättigten Gruppe zwischen dem Phenylkern und der Acylguanidingruppe beschrieben oder nahegelegt.

Es war überraschend, daß die erfindungsgemäßen Verbindungen keine unerwünschten und nachteiligen salidiuretischen, jedoch sehr gute antiarrhythmische Eigenschaften aufweisen; sie sind deshalb gut geeignet zum Behandeln von Zuständen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ -Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na ⁺ /H ⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- El: electron impact
- DCI: Desorption-Chemical Ionisation
- RT: Raumtemperatur
- EE: Ethylacetat (EtOAc)
- mp: Schmelzpunkt
- HEP: n-Heptan
- DME: Dimethoxyethan
- ES: Elektrospray-lonisation
- FAB: Fast Atom Bombardment
- CH₂Cl₂: Dichlormethan
- THF: Tetrahydrofuran
- eq.: Äquivalent

### Experimenteller Teil

### Allgemeine Vorschriften zur Herstellung von Alkenylcarbonsäure-guanidinen (I)

### Variante A: aus Alkenylcarbonsäuren (II, L = OH)

1.0 eq. des Carbonsäurederivates der Formel II löst bzw. suspendiert man in wasserfreiem THF ( 5 ml/mmol ) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2N HCI auf pH 6 bis 7 und filtriert das entsprechende Guanidid (Formel I) ab. Die so erhaltenen Carbonsäureguanidine können durch Behandeln mit wäßriger, methanolischer oder etherischer Salzsäure oder anderen pharmakologisch verträglichen Säuren in die entsprechenden Salze übergeführt werden.

### Variante B: aus Alkenylcarbonsäure-alkylestern (II, L = O-Alkyl)

1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, der Rückstand wird in EE aufgenommen, und diese Lösung wird und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert. (Salzbildung vergleiche Variante A)

### Beispiel 1: meta-Trifluormethylzimtsäureguanidid hydrochlorid

Wurde gemäß Variante A ausgehend von meta-Trifluormethylzimtsäure dargestellt.
Farblose Kristalle
mp 182 - 190°C.

### Beispiel 2: ortho-Trifluormethylzimtsäureguanidid hydrochlorid

Wurde gemäß Variante A ausgehend von ortho-Trifluormethylzimtsäure dargestellt.
Farblose Kristalle
mp 185 - 200°C.

### Beispiel 3: trans-2-Methyl-3-(3-trifluormethylphenyl)-acrylsäureguanidid

3 a) 1 eq. 2-Phosphonopropionsäuretriethylester wurde bei 0°C mit 1 eq. n-Butyllithium in Hexan deprotoniert und anschließend bei RT mit 1 eq. Trifluormethylbenzaldehyd versetzt. Nach vollständiger Abreaktion des Aldehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte trans-2-Methyl-3-(3-trifluormethylphenyl)-acrylsäureethylester ( < 3% mit dem cis-lsomeren verunreinigt).
3b) Der Ester aus 3 a) wurde gemäß Variante B zum trans-2-Methyl-3-(3-trifluor-methylphenyl)-acrylsäureguanidid umgesetzt.
   MS : 272 (M + 1)⁺
   mp: 124 - 126°C

### Beispiel 4: trans-2-Methyl-3-[3,5-Bis-(trifluormethyl)-phenyl]-acrylsäureguanidid-hydrochlorid

Wurde in Analogie zu Beispiel 3 aus 3,5-Bis-(trifluormethyl)-benzaldehyd dargestellt.
MS : 340 (M + 1)⁺
mp: 56 - 58°C

### Beispiel 5: trans-2-Methyl-3-[2-fluor-5-trifluormethyl-phenyl]-acrylsäureguanidid

Wurde in Analogie zu Beispiel 3 aus 2-Fluor-5-trifluormethyl-benzaldehyd dargestellt.
MS : 290 (M + 1)⁺
mp: 139°C

### Beispiel 6: trans-2-Methyl-3-[2-chlor-5-trifluormethyl-phenyl]-acrylsäureguanidid

Wurde in Analogie zu Beispiel 3 aus 2-Chlor-5-trifluormethyl-benzaldehyd dargestellt.
MS : 306 (M + 1)⁺
mp: 124 - 132°C

### Beispiel 7: trans-2-Methyl-3-[3-fluor-5-trifluormethyl-phenyl]-acrylsäureguanidid

Wurde in Analogie zu Beispiel 3 aus 3-Fluor-5-trifluormethyl-benzaldehyd dargestellt.
MS : 290 (M + 1)⁺
mp: 132°C

### Beispiel 8: trans-2-Methyl-3-[2-fluor-6-trifluormethyl-phenyl]-acrylsäureguanidid-hydrochlorid

Wurde in Analogie zu Beispiel 3 aus 2-Fluor-6-trifluormethyl-benzaldehyd dargestellt.
MS : 290 (M + 1)⁺
mp: 95°C

### Beispiel 9: trans-2-Methyl-3-[2-trifluormethyl-phenyl]-acrylsäureguanidid

Wurde in Analogie zu Beispiel 3 aus 2-Trifluormethyl-benzaldehyd dargestellt.
MS : 272 (M + 1)⁺
mp: 124 - 130 °C

### Beispiel 10: trans-2,3-Dimethyl-3-[2-fluor-5-trifluormethyl-phenyl]-acrylsäureguanidid

wurde in Analogie zu Beispiel 3 aus 2-Fluor-5-trifluormethyl-acetophenon hergestellt.
MS: 304 (M + 1)⁺
mp: 189°C

### Beispiel 11: cis-2,3-Dimethyl-3-[2-fluor-5-trifluormethyl-phenyl]-acrylsäureguanidid

wurde in Analogie zu Beispiel 3 aus 2-Fluor-5-trifluormethyl-acetophenon hergestellt.
MS: 304 (M + 1)⁺
mp: 140°C

### Beispiel 12: trans-2,3-Dimethyl-3-[3-trifluormethyl-phenyl]-acrylsäureguanidid

wurde in Analogie zu Beispiel 3 aus 2-Fluor-5-trifluormethyl-acetophenon hergestellt.
MS: 286 (M + 1)⁺
mp: 129°C

### Beispiel 13: trans-2-Methyl-3-trifluormethyl-3-[3-trifluormethyl-phenyl]-acrylsäure-guanidid

wurde in Analogie zu Beispiel 3 aus ωωω-Trifluormethyl-3-trifluormethylacetophenon hergestellt.
MS: 340 (M + 1)⁺
mp: 63°C

### Beispiel 14: trans-2-Trifluormethyl-3-phenyl-acrylsäure-guanidid

wurde gemäß Variante A aus cis-2-Trifluormethylzimtsäure (nach literaturbekannten Verfahren dargestellt) hergestellt.
MS: 258 (M + 1)⁺
amorph

### Beispiel 15 : Z-2-Fluor-3-(2-fluor-5-trifluormethyl-phenyl)propensäureguanidid Hydrochlorid

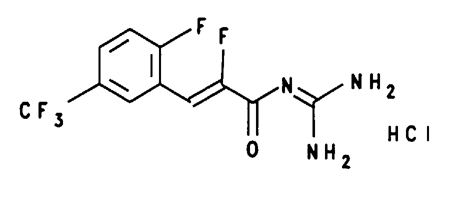

15a) Z-2-Fluor-3-(2-fluor-5-trifluormethyl-phenyl)propensäureethylester wurde in Analogie zu Beispiel 3 aus Triethyl-2-fluor-2-phosphonat und 2-Fluor-5-trifluormethyl-benzaldehyd dargestellt. Die anfallenden Doppelbindungsisomeren wurden chromatographisch getrennt.
15b) Der Z-Ester wurde unter Standardbedingungen mit Natriumhydroxid in Methanol zur freien Carbonsäure verseift und aufgearbeitet.
15c) Z-2-Fluor-3-(2-fluor-5-trifluormethyl-phenyl)propensäure wurde gemäß Variante A zunächst ins Propensäureguanidid und anschließend ins Guanididhydrochlorid überführt.
   MS: 294 (M + 1)⁺
   mp: 165°C (freie Base, Hydrochlorid amorph)

### Pharmakologische Daten:

Inhibition des Na⁺/H⁺-Exchangers von Kaninchenerythozyten:
Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na⁺-Ausgangsgehalts der Erythrozyten.

Um den Amilorid-sensitiven Natrium-lnflux zu bestimmen, wurden 100 µl jeder Blutprobe in jeweils 5 ml eines hyperosmolaren Salz-Sucrose-Mediums (mmol/l: 140 NaCl, 3 KCI, 150 Sucrose, 0,1 Ouabain, 20 Tris-hydroxymethylaminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrozyten nach Inkubation errechnet. Der Amilorid-hemmbare Natrium-lnflux ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

Ergebnisse zur Inhibition des Na⁺/H⁺-Exchangers:

| Beispiel ( siehe exp. Teil ) | IC₅₀ (µmol) |
|---|---|
| 1 | <1 |
| 3 | <1 |
| 10 | <1 |

## Patentansprüche

1. Perfluoralkylgruppen tragende phenylsubstituierte Alkenylcarbonsäure-guanidide der Formel I 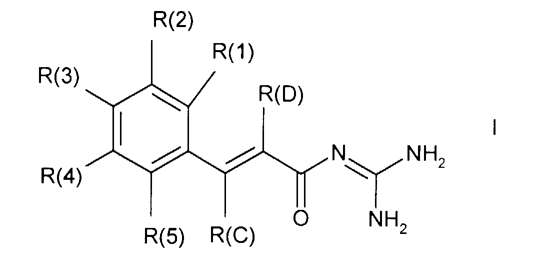 worin bedeuten:
R(C) Wasserstoff, F, Cl, Br, I, CN, (C₁-C₈)-Alkyl_{,} C_{b}F_{2b+1};
b 1, 2, 3, 4, 5, 6, 7 oder 8;
R(D) unabhängig wie R(C) definiert,
R(1) Wasserstoff, (C₁-C₄)-Alkyl, C_{b}F_{2b+1}, (C₃-C₈)-Cycloalkyl, F, Cl, Br, I oder CN;
b 1, 2, 3 oder 4;
R(2), R(3), R(4) und R(5) unabhängig voneinander wie R(1) definiert;
jedoch unter der Bedingung,
daß mindestens einer der Substituenten R(C), R(D), R(1), R(2), R(4) oder R(5) eine C_{b}F_{2b+1}-Gruppe, und daß R(3) keine C_{b}F_{2b+1}-Gruppe ist.

2. Verfahren zur Herstellung einer Verbindung I nach Anspruch 1, dadurch gekennzeichnet, daß man
eine Verbindung der Formel II 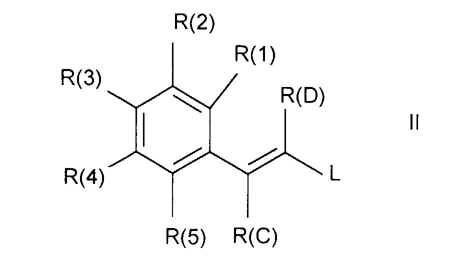 mit Guanidin umsetzt, worin R(1) bis R(5), R(C) und R(D) die angegebene Bedeutung besitzen und L für eine leicht nucleophil substituierbare leaving group steht.

3. Verwendung einer Verbindung I nach Anspruch 1 zur Herstellung eines Medikaments zu Behandlung von Arrhythmien.

4. Verwendung einer Verbindung I nach Anspruch 1 zur Herstellung eines Medikaments zur Behandlung oder Prophylaxe des Herzinfarktes.

5. Verwendung einer Verbindung I nach Anspruch 1 zur Herstellung eines Medikaments zur Behandlung oder Prophylaxe der Angina Pectoris.

6. Verwendung einer Verbindung I nach Anspruch 1 zur Herstellung eines Medikaments zur Behandlung oder Prophylaxe von ischämischen Zuständen des Herzens.

7. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

8. Verwendung einer Verbindung I nach Anspruch 1 zur Herstellung eines Medikaments zur Behandlung oder Prophylaxe von ischämischen Zuständen peripherer Organe und Gliedmaßen.

9. Verwendung einer Verbindung I nach Anspruch 1 zur Herstellung eines Medikaments zur Behandlung von Schockzuständen.

10. Verwendung einer Verbindung I nach Anspruch 1 zur Herstellung eines Medikaments zum Einsatz bei chirurgischen Operationen und Organtransplantationen.

11. Verwendung einer Verbindung I nach Anspruch 1 zur Herstellung eines Medikaments zur Konservierung und Lagerung von Transplantaten für chirurgische Maßnahmen.

12. Verwendung einer Verbindung I nach Anspruch I zur Herstellung eines Medikaments zur Behandlung von Krankheiten, bei denen die Zellproliferation eine primäre oder sekundäre Ursache darstellt:

13. Heilmittel, gekennzeichnet durch einen wirksamen Gehalt einer Verbindung der Formel I nach einem oder mehreren der Ansprüche 1 bis 3.

## Claims

1. A phenyl-substituted alkenylcarboguanidide carrying perfluoroalkyl groups, of the formula I 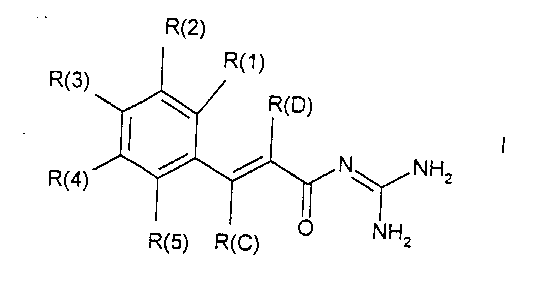 in which
R(C) is hydrogen, F, Cl, Br, I, CN, (C₁-C₈)-alkyl, C_{b}F_{2b+1};
b is 1, 2, 3, 4, 5, 6, 7 or 8;
R(D) is defined independently as R(C),
R(1) is hydrogen, (C₁-C₄)-alkyl, C_{b}F_{2b+1}, (C₃-C₈)-cycloalkyl, F, Cl, Br, I or CN;
b is 1, 2, 3 or 4;
R(2), R(3), R(4) and R(5) are defined independently of one another as R(1);
but with the condition
that at least one of the substituents R(C), R(D), R(1), R(2), R(4) or R(5) is a C_{b}F_{2b+1} group, and that R(3) is not a C_{b}F_{2b+1} group.

2. A process for the preparation of a compound I as claimed in claim 1, which comprises reacting a compound of the formula II 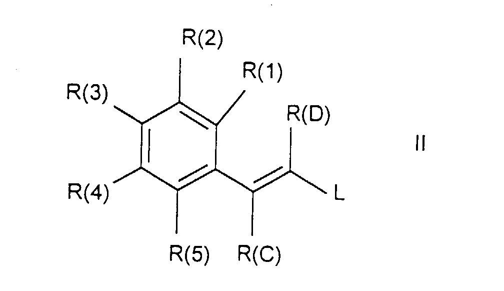 with guanidine, in which R(1) to R(5), R(C) and R(D) have the stated meaning, and L is a leaving group which easily undergoes nucleophilic substitution.

3. The use of a compound I as claimed in claim 1 for the preparation of a medicament for the treatment of arrhythmias.

4. The use of a compound I as claimed in claim 1 for the preparation of a medicament for the treatment or prophylaxis of myocardial infarct.

5. The use of a compound I as claimed in claim 1 for the preparation of a medicament for the treatment or prophylaxis of angina pectoris.

6. The use of a compound I as claimed in claim 1 for the preparation of a medicament for the treatment or prophylaxis of ischemic states of the heart.

7. The use of a compound I as claimed in claim 1 for the preparation of a medicament for the treatment or prophylaxis of ischemic states of the peripheral and central nervous system and of stroke.

8. The use of a compound I as claimed in claim 1 for the preparation of a medicament for the treatment or prophylaxis of ischemic states of the peripheral organs and limbs.

9. The use of a compound I as claimed in claim 1 for the preparation of a medicament for the treatment of states of shock.

10. The use of a compound I as claimed in claim 1 for the preparation of a medicament for use in surgical operations and organ transplants.

11. The use of a compound I as claimed in claim 1 for the preparation of a medicament for the preservation and storage of transplants for surgical procedures.

12. The use of a compound I as claimed in claim 1 for the preparation of a medicament for the treatment of diseases in which cell proliferation represents a primary or secondary cause.

13. A medicine which has an effective content of a compound of the formula I as claimed in one or more of claims 1 to 3.

## Revendications

1. Guanidides d'acides alcénylcarboxyliques substitués par un phényle et portant des groupes perfluoroalkyle de formule I: 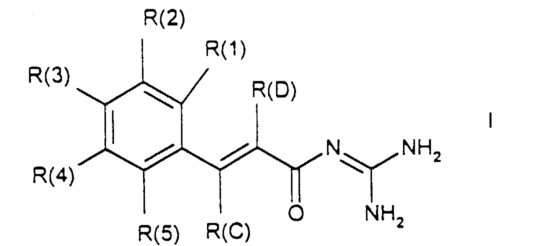 dans laquelle :
R(C) représente hydrogène, F, Cl, Br, I, CN, alkyle en C₁-C₈, C_{b}F_{2b+1} ;
b est 1, 2, 3, 4, 5, 6, 7 ou 8 ;
R(D) est indépendamment tel que défini pour R(C),
R(1) représente hydrogène, alkyle en C₁-C₄, C_{b}F_{2b+1}, cycloalkyle en C₃-C₈, F, Cl, Br, I ou CN ;
b est 1, 2, 3 ou 4 ;
R(2), R(3), R(4) et R(5) sont, indépendamment les uns des autres, tels que défini pour R(1) ;
à la condition cependant qu'au moins un des substituants R(C), R(D), R(1), R(2), R(4) ou R(5) représente un groupe C_{b}F_{2b+1} et que R(3) ne représente pas un groupe C_{b}F_{2b+1}.

2. Procédé de préparation d'un composé I selon la revendication 1, caractérisé en ce que :
on fait réagir, avec de la guanidine, un comDosé de formule II 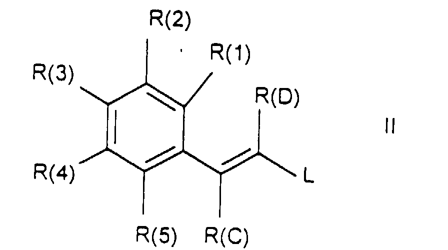 dans laquelle R(1) à R(5), R(C) et R(D) ont les significations indiquées et L est un groupe partant qui peut être facilement substitué par un nucléophile.

3. Utilisation d'un composé I selon la revendication 1 pour la préparation d'un médicament pour le traitement des arythmies.

4. Utilisation d'un composé I selon la revendication 1 pour la préparation d'un médicament pour le traitement ou la prophylaxie des infarctus cardiaques.

5. Utilisation d'un composé I selon la revendication 1 pour la préparation d'un médicament pour le traitement ou la prophylaxie de l'angine de poitrine.

6. Utilisation d'un composé I selon la revendication 1 pour la préparation d'un médicament pour le traitement ou la prophylaxie des états ischémiques du coeur.

7. Utilisation d'un composé I selon la revendication 1 pour la préparation d'un médicament pour le traitement ou la prophylaxie des états ischémiques du système nerveux périphérique et central et des états ischémiques dus à une attaque.

8. Utilisation d'un composé I selon la revendication 1 pour la préparation d'un médicament pour le traitement ou la prophylaxie des états ischémiques des organes périphériques et des membres.

9. Utilisation d'un composé I selon la revendication 1 pour la préparation d'un médicament pour le traitement des états de choc.

10. Utilisation d'un composé I selon la revendication 1 pour la préparation d'un médicament pour une utilisation lors des opérations chirurgicales et des transplantations d'organes.

11. Utilisation d'un composé I selon la revendication 1 pour la préparation d'un médicament pour la conservation et le stockage des transplants pour des interventions chirurgicales.

12. Utilisation d'un composé I selon la revendication 1 pour la préparation d'un médicament pour le traitement des maladies, dans lesquelles la prolifération cellulaire est un effet primaire ou secondaire.

13. Agent thérapeutique caractérisé par une quantité efficace d'un composé I selon une ou plusieurs des revendications 1 à 3.
